# EUROPEAN PATENT SPECIFICATION

(11) **EP 4 658 218 B1**
(45) Date of publication and mention of the grant of the patent: **14.10.2026**
(21) Application number: 24715677.1
(22) Date of filing: 27.02.2024
(51) Int. Cl.: A61F 13/20, A61B 5/00, A61F 5/44, A61F 13/42, A61F 13/45

(54) **INTRAVAGINAL DEVICE WITH EXPANDABLE PORTION**
INTRAVAGINALE VORRICHTUNG MIT EXPANDIERBAREM TEIL
DISPOSITIF INTRAVAGINAL AVEC PARTIE EXTENSIBLE

(30) Priority: 27.02.2023 US 202363487016 P
(43) Date of publication of application: 10.12.2025
(73) Proprietor: Gals Bio Ltd., 9079265 Mevaseret Zion (IL)
(72) Inventor: SHAVIV, Hilla, 9079265 Mevaseret Zion (IL); SHEZIFI, Omer, 3479514 Haifa (IL); AGRON, Raz, 9666452 Jerusalem (IL); KINGMA, Rimer, 3604467 Kiryat Tivon (IL)
(74) Representative: Jaeger, Michael David
(86) International application number: PCT/IB2024/051865
(87) International publication number: WO 2024/180473

(56) References cited:
- EP-A1- 3 377 369
- EP-A1- 3 773 369
- WO-A1-2018/100213
- CN-A- 108 784 911
- US-A1- 2016 220 342
- US-A1- 2017 231 831

## Description

### FIELD OF THE INVENTION

The present invention relates to an intravaginal device, and particularly to an intravaginal device with an expandable portion which can be used to collect therein vaginal effluents.

### BACKGROUND OF THE INVENTION

European Patent 3377369 describes an intravaginal device includes arms and struts. Each strut has a first end coupled to a shaft arranged to slide in a tube and a second end coupled to one of the arms. A string is coupled to the shaft. The arms have a stowed orientation in which the struts are folded inwards and a deployed orientation in which the struts point radially outwards and tautly hold the arms in an expanded position.

European Patent 3377369 is implemented in the device TULIPON of Gals Bio Ltd., Israel. This device is a disposable, or partly disposable and partly reusable, vaginal platform used like a regular tampon with an applicator that collects the vaginal effluents better, safer, and longer with higher capacity than any other tampon.

### SUMMARY

The present invention seeks to provide an intravaginal device with an expandable portion, which has added features not found in European Patent 3377369. The invention is defined by the claims provided herein.

The intravaginal device can be fully disposable or partly disposable, partly reusable or fully reusable.

There is provided an intravaginal device including an expandable portion including one or more struts pivotally coupled to one or more link arms, the one or more link arms being pivotally coupled to a shaft, and the one or more struts being pivotally coupled to a base member coupled to the shaft, a strut expansion mechanism coupled to the one or more struts for expanding the one or more struts outwards from the shaft, and a pull string coupled to a portion of the expandable portion.

In accordance with the invention the one or more struts include a greater expanding strut group and a lesser expanding strut group, wherein the greater expanding strut group expands further outwards than the lesser expanding strut group.

In accordance with a non-limiting embodiment of the invention the one or more struts include a greater expanding strut group and a lesser expanding strut group, wherein the greater expanding strut group slides a longer distance on the shaft than the lesser expanding strut group.

In accordance with a non-limiting embodiment of the invention the one or more struts include a plurality of struts that expand outwards in non-uniform expansion to a non-circular shape.

In accordance with a non-limiting embodiment of the invention the strut expansion mechanism includes a first sliding ring pivotally coupled to the greater expanding strut group and arranged to slide on the shaft, and a second sliding ring pivotally coupled to the lesser expanding strut group and arranged to slide on the shaft.

In accordance with a non-limiting embodiment of the invention first sliding ring includes a lug received in a channel formed in the second sliding ring.

In accordance with a non-limiting embodiment of the invention the intravaginal includes a vaginal effluent collection bag.

In accordance with a non-limiting embodiment of the invention the expandable portion is initially inside a removable cover.

In accordance with a non-limiting embodiment of the invention the expandable portion includes one or more sensors and other electrical components.

In accordance with a non-limiting embodiment of the invention the one or more sensors and other electrical components include at least one of: a transmitting component, a receiving component, an ultrasonic transducer, a sonic transducer, a volumetric and/or fluid flow sensor, an optical sensor, a Doppler sensor, a camera, a light emitting source, and a lateral flow test sensor.

In accordance with a non-limiting embodiment of the invention the expandable portion is initially inside an applicator including an openable distal end formed with slits, and a plunger is arranged to move with respect to the applicator which causes relative movement of the expandable portion with respect to the applicator.

In accordance with a non-limiting embodiment of the invention the bag fully encapsulates the device to enable collection of any fluids.

In accordance with a non-limiting embodiment of the invention the bag includes an anti-bacterial material or has a texture which has anti-bacterial properties.

In accordance with a non-limiting embodiment of the invention the bag is connected to the pull string via a closing valve and when the string is pulled the valve closes the bag.

### BRIEF DESCRIPTION OF DRAWINGS

The present invention will be understood and appreciated more fully from the following detailed description, taken in conjunction with the drawings in which:
Fig. 1 is a simplified illustration of an intravaginal device, in accordance with a non-limiting embodiment of the present invention.
Fig. 2 is a simplified illustration of the intravaginal device being inserted in a vagina.
Fig. 3 is a simplified illustration of starting to retract an applicator of the intravaginal device, which starts to expose an expandable portion.
Fig. 4 is a simplified, cutaway illustration of the intravaginal device, with the expandable portion inside the applicator (as in Figs. 1 and 2).
Fig. 5 is a simplified, cutaway illustration of the intravaginal device, with the applicator partially retracted from the expandable portion (as in Fig. 3), and the expandable portion not yet expanded.
Figs. 5A and 5B are enlarged illustrations of the proximal and distal portions of the device, respectively, shown in Fig. 5.
Fig. 6 is a simplified, cutaway illustration of the intravaginal device, with the applicator partially retracted from the expandable portion, and the expandable portion partially expanded.
Fig. 6A is an enlarged illustration of the proximal portion of the device, showing a locking mechanism for maintaining the expandable portion partially expanded.
Fig. 7 is a simplified, cutaway illustration of the intravaginal device, with the applicator fully retracted from the expandable portion, and the expandable portion is almost fully expanded.
Fig. 8 is a simplified, cutaway illustration of the intravaginal device, with the applicator starting to be removed from the device, and the expandable portion is fully expanded.
Fig. 9 is a simplified illustration of the intravaginal device in the vagina with the applicator removed, thereby exposing a pull string, and the expandable portion fully expanded.
Fig. 10 is a simplified illustration of a strut expansion mechanism of the intravaginal device, in accordance with a non-limiting embodiment of the present invention, in which one group of struts is expanded more radially outwards than another group of struts, by using a sliding ring for the less-expanded group of struts.
Fig. 11 is a simplified illustration of the one group of struts expanded more radially outwards than the other group of struts.
Fig. 12 is a simplified illustration of a strut expansion mechanism of the intravaginal device, in accordance with another non-limiting embodiment of the present invention, in which a link arm of one group of struts is positioned to cause greater radially outward expansion than another link arm of the other group of struts.
Figs. 13 and 14 are simplified illustrations of the intravaginal device in the expanded orientation, respectively with and without a vaginal effluent collection bag, showing that with the non-uniform expansion of the struts, the device conforms to the curved and non-circular shape of the vaginal or cervical anatomical shape.
Figs. 15A and 15B are simplified illustrations of an intravaginal device, in accordance with two non-limiting embodiments of the present invention, which include sensors and/or other electrical components for sensing, monitoring and communicating (both transmission and reception) data related to the vaginal environment.

### DETAILED DESCRIPTION

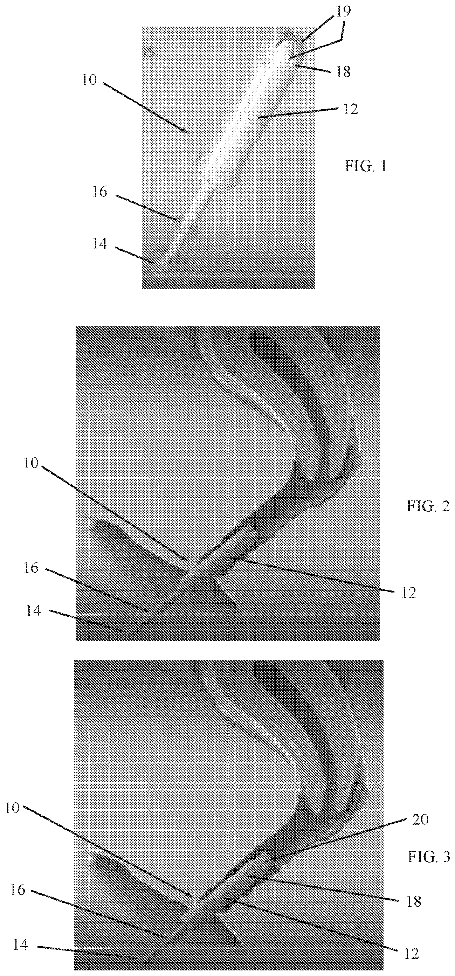

Reference is now made to Figs. 1-3, which illustrate an intravaginal device 10, in accordance with a non-limiting embodiment of the present invention.

The intravaginal device 10 as seen in Fig. 1 has an outer applicator 12, which initially covers the inner operative elements of the device. The device 10 may include a plunger 14 which causes relative movement of the inner operative elements of the device with respect to applicator 12 (as described below), and a handle 16. The applicator 12 may include an openable distal end 18, which may be formed with slits 19. This allows the distal end of the applicator 12 to open sufficiently to allow passage therethrough of the expandable portion of the device, as described below.

Fig. 2 illustrates the intravaginal device 10 being inserted in a vagina.

Fig. 3 illustrates starting to retract applicator 12, which starts to expose an expandable portion 20, which is described in detail below.

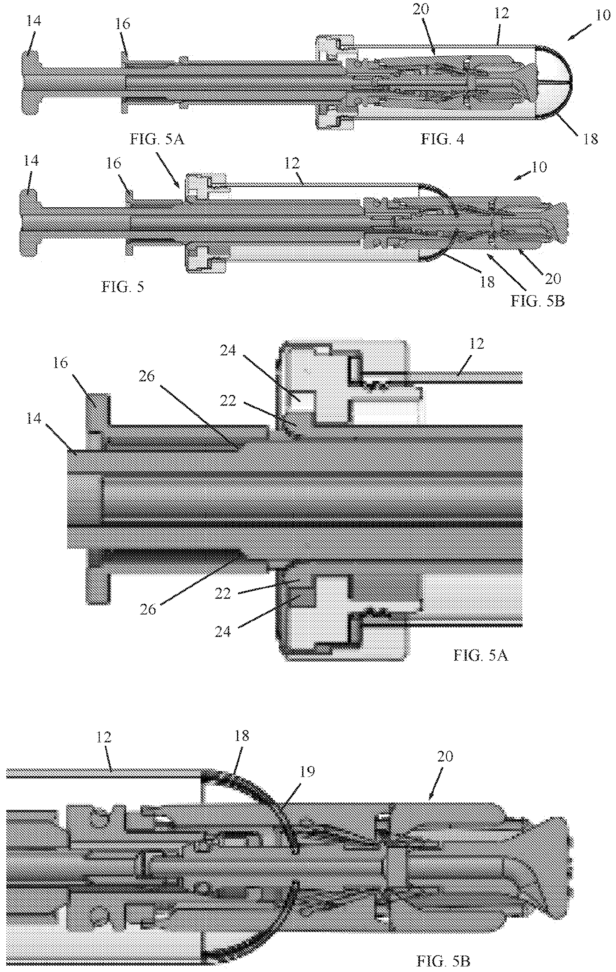

Fig. 4 illustrates the intravaginal device 10, with the expandable portion 20 inside the applicator 12.

Reference is now made to Fig. 5, which illustrates the intravaginal device 10, with plunger 14 moved distally (to the right in the sense of the drawing), thereby moving expandable portion 20 distally. This movement causes the applicator 12 to be partially retracted from the expandable portion 20 (as in Fig. 3). The expandable portion 20 is not yet expanded.

As seen in Fig. 5A, at this position of Fig. 5, a distal rim 22 is received in a groove 24 (such as an annular groove) formed in a distal portion of applicator 12. As long as rim 22 is received in groove 24, the position of applicator 12 relative to the rest of the device is fixed.

Fig. 5B is an enlargement showing the expandable portion 20 protruding distally through the slits 19 of openable distal end 18 of the applicator 12.

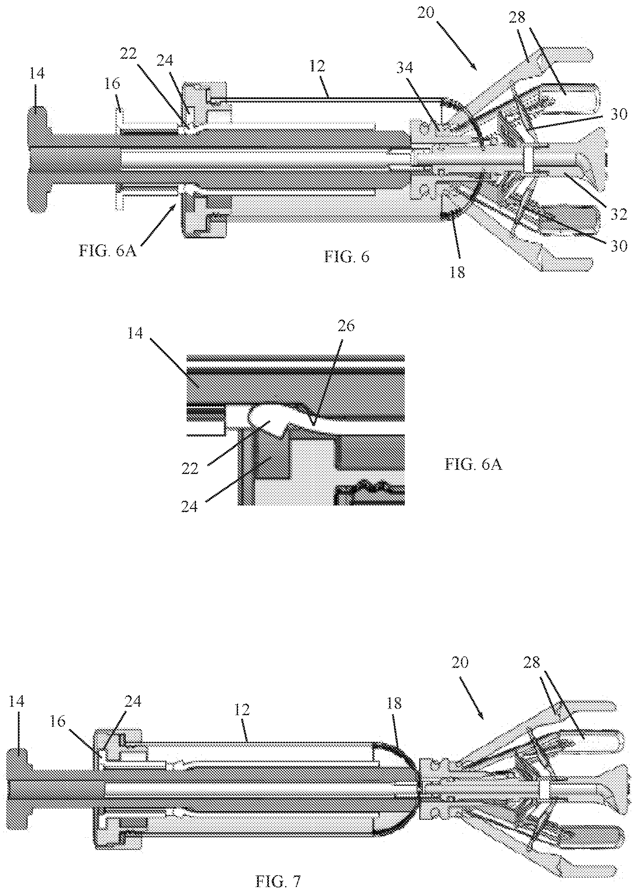

Reference is now made to Fig. 6, which illustrates the expandable portion 20 partially retracted from the applicator 12. This is caused by further distal movement of the plunger 14, which dislodges rim 22 out of groove 24. Rim 24 bends or otherwise deforms and now abuts against a depression 26 (e.g., an annular depression) formed in plunger 14, as seen best in Fig. 6A. In this position, handle 16 can continue to move together with plunger 14 against applicator 12 so as to further expose expandable portion 20 that is right now partially expanded. The movement of handle 16 is possible until handle 16 reaches and is stopped in groove 24, and expandable portion 20 can expand further (as described below with reference to Fig. 7).

In Fig. 6, it is seen that the expandable portion 20 may include one or more struts 28 pivotally coupled to one or more link arms 30. Link arms 30 may be pivotally coupled to a shaft 32. The struts 28 may be pivotally coupled to a base member 34 coupled to shaft 32. In the position of Fig. 6, the expandable portion 20 is only partially expanded because the slits 19 of the distal end 18 only permit partial outward expansion of struts 28 and not complete outward expansion.

Reference is now made to Fig. 7. Handle 16 has been moved distally and is received in groove 24. The expandable portion 20 is now fully deployed out of applicator 12. This permits the struts 28 of expandable portion 20 to be almost fully expanded.

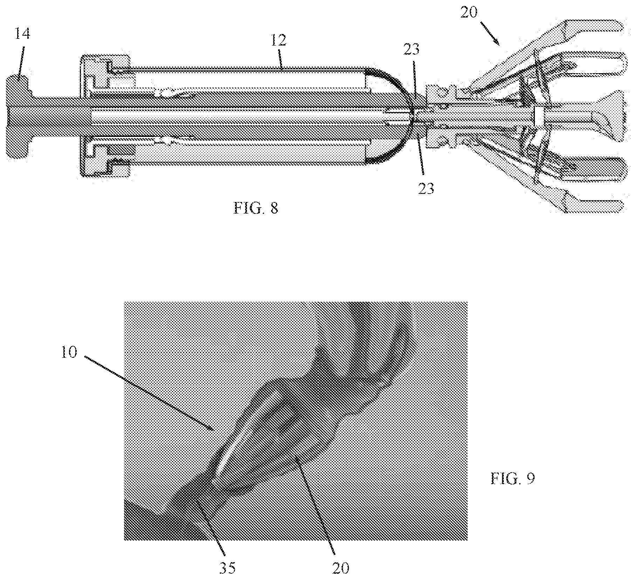

Reference is now made to Fig. 8. The expandable portion 20 is now fully expanded but still coupled to the plunger 14 by means of fingers 23 at a distal end of plunger 14 that grasp expandable portion 20. Further distal movement of plunger 14 releases fingers 23 from expandable portion 20. Once expandable portion 20 is released from plunger 14, the applicator 12 and plunger 14 can be retracted proximally away from the vaginal cavity while leaving the device (which is now expandable portion 20 without the applicator and plunger) fully deployed as in Fig. 9.

Reference is now made to Fig. 9, which illustrates the intravaginal device 10 in the vagina with the applicator removed, thereby exposing a pull string 35. The expandable portion 20 is fully expanded. The pull string 35 can be used to pull out the intravaginal device 10 from the vagina after the device has been used to collect vaginal effluents.

The structure described above with reference to Figs. 1-9, enhances the ability of the device 10 to operate efficiently with a variety of vaginal anatomies and cervical positions. As described above, the first stage in opening the device 10 is a limited opening (without limitation, for example, about 30% to 60% of the final opening). This followed by further distal insertion (without limitation, for example, about 10 to 20 mm) into the vaginal canal while the expandable portion 20 continues its partial opening. The final stage is full expansion and release from the delivery mechanism.

As stated, one advantage of this structure is adaptability to a wide range of anatomies. By limiting the opening at the very starting point of the vaginal cavity and continued distal pushing while the device is kept in its limited opening stage. In this limited expansion, the device in most cases will not pass by the cervix opening (thereby minimizing or preventing any possible leakages) but will push the cervix, such as in cases of a tilted cervix, thereby keeping the collecting surface proximal to the cervix opening.

The inventors recognize that the vaginal canal is usual oval and not circular. This may be due pressure from the surrounding organs which is not symmetric. More forces tend to act against the vagina from the upper abdomen than the sides, thereby causing the flattened or oval shape of the vagina. The device 10 may thus be oval shaped to adapt to the vaginal shape and to reduce opening forces and pushing force from neighboring organs, thereby minimizing forces and potential damage to the vagina tissue.

The invention may provide structure to achieve the oval shape by providing different radial travel for each strut while keeping a similar linear travel, as is now described.

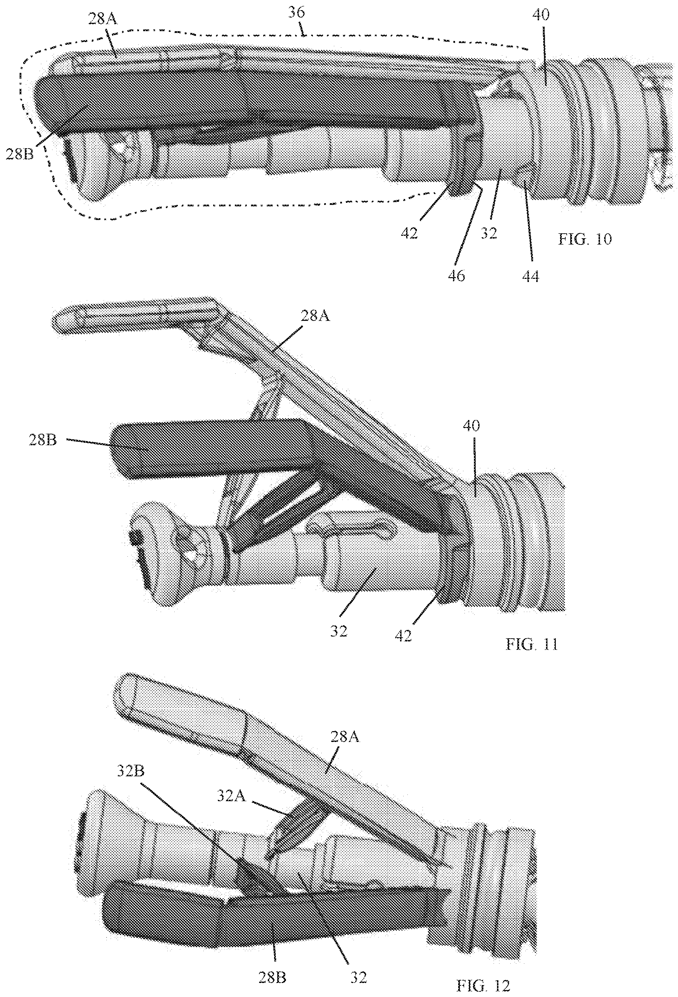

Reference is now made to Fig. 10, which illustrates a strut expansion mechanism of the intravaginal device, in accordance with a non-limiting embodiment of the present invention. In this embodiment, one group of struts (a "group" includes one or more struts), designated greater expanding strut group 28A, is expanded more radially outwards than another group of struts, designated lesser expanding strut group 28B.

The greater expanding strut group 28A may be pivotally coupled to a first sliding ring 40 arranged to slide on shaft 32. The lesser expanding strut group 28B may be pivotally coupled to a second sliding ring 42 on shaft 32. The first sliding ring 40 may have a lug 44 that can be received in a channel 46 formed in second sliding ring 42. The greater expanding group 28A slides a longer distance on shaft 32 than the lesser expanding group 28B. The movement of the groups may include initial linear movement of first sliding ring 40, then both rings 40 and 42 touch each other, followed by further movement of both rings 40 and 42 up to full opening of the expandable portion 20.

As seen by comparing Figs. 10 and 11, due to the sliding ring 40, the greater expanding strut group 28A has greater linear travel which causes greater radial expansion of the struts. This solution can also be used to compensate for any radial differences and tolerances in travel.

More than one sliding ring may be provided for more groups of differently expanding struts.

Reference is now made to Fig. 12, which illustrates a strut expansion mechanism of the intravaginal device, in accordance with another non-limiting embodiment of the present invention. In this embodiment, the link arm(s) 32A of the greater expanding strut group 28A is positioned to cause greater radially outward expansion than the link arm(s) 32B of the lesser expanding strut group 28B.

The embodiments of Figs. 10-12 can provide different side travels, expansion travel and even different lengths of the struts. Accordingly, the device 10 can open in a wide variety of shapes and geometries.

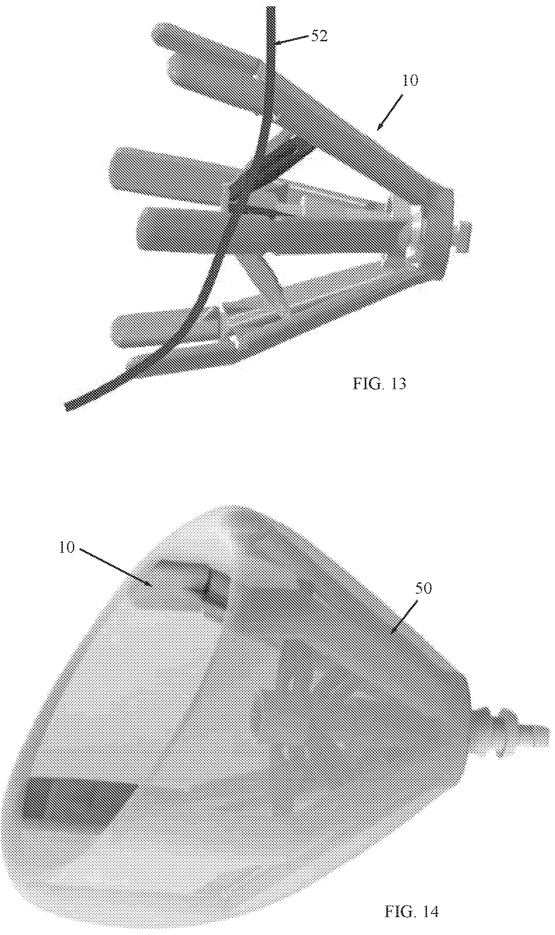

Reference is now made to Figs. 13 and 14, which illustration the intravaginal device 10 in the expanded orientation, respectively with and without a vaginal effluent collection bag 50. With the non-uniform expansion of the struts, the device 10 conforms to the curved and non-circular shape of the vaginal or cervical anatomical shape (the contour of this shape being designated by numeral 52 in Fig. 13).

It is important to note that the invention can be carried out without applicator 12. In another embodiment, there is no applicator and instead the device (expandable portion 20) is initially covered by a cover 36 (shown by broken lines in Fig. 10), such as but not limited to, nylon shrink or cup, which protects the device and is removed just before use. After removing the cover 36, the device may be inserted into the vagina. This embodiment has several advantages. It simplifies the design by reducing the number of parts. The lack of the applicator reduces the size (diameter) dramatically and also shortens the length of the unit which is inserted in the vagina. In another advantageous way of using the device, the device is inserted in the vagina and then the cover is gradually removed by pulling it proximally off the device and out of the vagina. This allows for partial expansion as described above. In any case, no matter when the cover is removed, the expandable portion 20 expands outwards as described above.

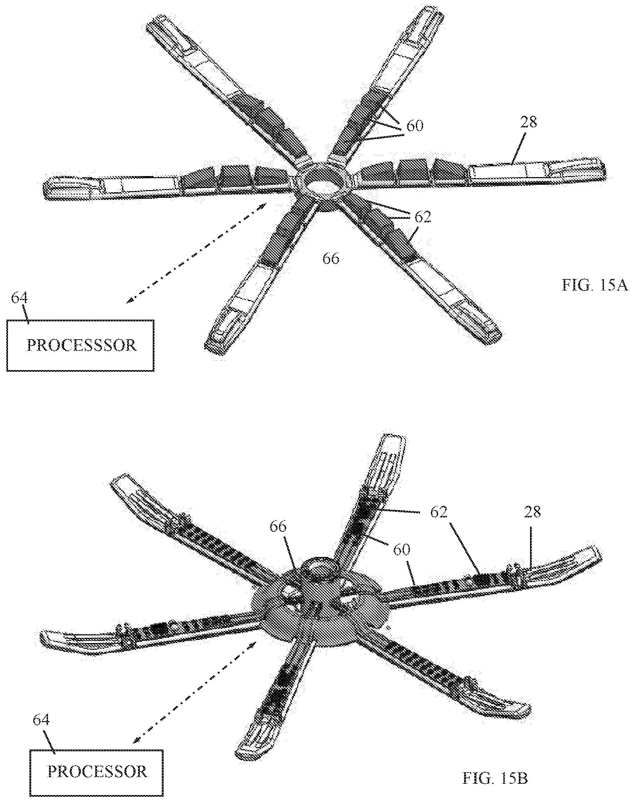

Reference is now made to Figs. 15A and 15B. In accordance with an embodiment of the invention, the device includes additional monitoring and treatment options.

Different sensors 60 and other electrical components 62 may be coupled to struts 28 or other portions of the device, such as for transmitting and receiving information to and from an external processor 64, which may be a handheld unit or a cloud-based processor or other kind of processor or controller. These sensors and components may be energized by a battery 66, which may be located in the device, such as on a central ring, or may be energized from an external source by wireless means.

Non-limiting examples of sensors include an ultrasonic transducer that can perform a partial or complete peripheral scan while staying in the vaginal cavity; volumetric and/or fluid flow sensors that can measure the volume and flow of the vaginal fluid (e.g., optical sensor, inductive sensor, float or other mechanical sensor, and many more); optical sensors operating at certain wavelengths to measure the level of oxidation or other parameter in the vaginal environment; a Doppler sensor to test and measure the flow in the vagina; a camera operating at different wavelengths to examine the vaginal environment.

The electrical components may be used to provide treatment to the vaginal environment. Without limitation, examples include using blue wavelength light in combination with frequencies to treat various kinds of fungi; laser light of a suitable wavelength for gentle burning of tissue and/or rejuvenation of the vaginal cavity.

The device may perform preliminary actions on the liquid collected therein. For example, the device may include a filter for filtering the collected liquid to filter out tissue lumps, clots and other debris. Alternatively, since the liquid is released slowly over a long period of time, it may be desirable to leave the homogeneity of the liquid over time without filtering in order to reflect an average. Alternatively, mixing and sampling may be done at different time intervals over the entire use. Initial filtration of the liquid may be done by very fine filtration, or by magnetic fields or other means.

## Claims

1. An intravaginal device (10) comprising:
an expandable portion (20) comprising one or more struts (28) pivotally coupled to one or more link arms (30), said one or more link arms (30) being pivotally coupled to a shaft (32), and said one or more struts (28) being pivotally coupled to a base member (34) coupled to said shaft (32), wherein said one or more struts (28) comprise a greater expanding strut group (28A) and a lesser expanding strut group (28B);
a strut expansion mechanism coupled to said one or more struts for expanding said one or more struts (28) outwards from said shaft (32), wherein said greater expanding strut group (28A) expands further outwards than said lesser expanding strut group (28B); and
a pull string (35) coupled to a portion of said expandable portion (20).

2. The intravaginal device according to claim 1, wherein the expansion mechanism is configured to slide the greater expanding strut group (28A) and the lesser expanding strut group (28B) on the shaft (32).

3. The intravaginal device according to claim 1 or 2, wherein said greater expanding strut group (28A) slides a longer distance on said shaft (32) than said lesser expanding strut group (28B).

4. The intravaginal device according to any one of claims 1-3, wherein said one or more struts (28) comprise a plurality of struts that expand outwards in non-uniform expansion to a non-circular shape.

5. The intravaginal device according to any one of claims 1-4, wherein said strut expansion mechanism comprises a first sliding ring (40) pivotally coupled to said greater expanding strut group (28A) and arranged to slide on said shaft (32), and a second sliding ring (42) pivotally coupled to said lesser expanding strut group (28B) and arranged to slide on said shaft (32).

6. The intravaginal device according to claim 5, wherein the first sliding ring (40) comprises a lug (44) received in a channel (46) formed in said second sliding ring (42).

7. The intravaginal device according to any one of claims 1-6, wherein said expandable portion (20) is initially inside a removable cover (36).

8. The intravaginal device according to any one of claims 1-7, wherein said expandable portion (20) comprises one or more sensors (60) and one or more other electrical components (62).

9. The intravaginal device according to any one of claims 1-8, wherein said expandable portion (20) comprises at least one of: a transmitting component, a receiving component, an ultrasonic transducer, a sonic transducer, a volumetric and/or fluid flow sensor, an optical sensor, a Doppler sensor, a camera, a light emitting source, and/or a lateral flow test sensor.

10. The intravaginal device according to any one of claims 1-9, wherein said expandable portion (20) is initially inside an applicator (12) comprising an openable distal end (18) formed with slits (19), and a plunger (14) is arranged to move with respect to said applicator (12) which causes relative movement of said expandable portion (20) with respect to said applicator (12).

11. The intravaginal device according to any one of claims 1-10, further comprising a vaginal effluent collection bag (50).

12. The intravaginal device according to claim 11, wherein said vaginal effluent collection bag (50) fully encapsulates the device to enable collection of any fluids.

13. The intravaginal device according to claim 11 or 12, wherein said vaginal effluent collection bag (50) comprises an anti-bacterial material or has a texture which has anti-bacterial properties.

14. The intravaginal device according to any one of claims 11-13, wherein said vaginal effluent collection bag (50) is connected to the pull string (35) via a closing valve and when the string (35) is pulled the valve closes said vaginal effluent collection bag (50).

## Patentansprüche

1. Intravaginale Vorrichtung (10), umfassend:
einen expandierbaren Abschnitt (20), der eine oder mehrere Streben (28) umfasst, die schwenkbar mit einem oder mehreren Verbindungsarmen (30) gekoppelt sind, wobei der eine oder die mehreren Verbindungsarme (30) schwenkbar mit einer Welle (32) gekoppelt sind und die eine oder die mehreren Streben (28) schwenkbar mit einem mit der Welle (32) gekoppelten Basiselement (34) gekoppelt sind, wobei die eine oder die mehreren Streben (28) eine Gruppe stärker expandierender Streben (28A) und eine Gruppe weniger stark expandierender Streben (28B) umfassen;
einen mit der einen oder den mehreren Streben gekoppelten Strebenexpansionsmechanismus zum Expandieren der einen oder mehreren Streben (28) von der Welle (32) nach außen, wobei die Gruppe stärker expandierender Streben (28A) weiter nach außen expandiert als die Gruppe weniger stark expandierender Streben (28B); und
eine Zugschnur (35), die mit einem Abschnitt des expandierbaren Abschnitts (20) gekoppelt ist.

2. Intravaginale Vorrichtung nach Anspruch 1, wobei der Expansionsmechanismus dazu konfiguriert ist, die Gruppe stärker expandierender Streben (28A) und die Gruppe weniger stark expandierender Streben (28B) auf der Welle (32) zu verschieben.

3. Intravaginale Vorrichtung nach Anspruch 1 oder 2, wobei sich die Gruppe stärker expandierender Streben (28A) über eine längere Strecke auf der Welle (32) verschiebt als die Gruppe weniger stark expandierender Streben (28B).

4. Intravaginale Vorrichtung nach einem der Ansprüche 1-3, wobei die eine oder mehreren Streben (28) eine Vielzahl von Streben umfassen, die in einer ungleichmäßigen Expansion nach außen zu einer nicht kreisförmigen Form expandieren.

5. Intravaginale Vorrichtung nach einem der Ansprüche 1-4, wobei der Strebenexpansionsmechanismus einen ersten Verschiebering (40), der schwenkbar mit der Gruppe stärker expandierender Streben (28A) gekoppelt und dazu angeordnet ist, sich auf der Welle (32) zu verschieben, und einen zweiten Verschiebering (42), der schwenkbar mit der Gruppe weniger stark expandierender Streben (28B) gekoppelt und dazu angeordnet ist, sich auf der Welle (32) zu verschieben, umfasst.

6. Intravaginale Vorrichtung nach Anspruch 5, wobei der erste Verschiebering (40) einen Vorsprung (44) umfasst, der in einem in dem zweiten Verschiebering (42) ausgebildeten Kanal (46) aufgenommen ist.

7. Intravaginale Vorrichtung nach einem der Ansprüche 1-6, wobei sich der expandierbare Abschnitt (20) anfänglich innerhalb einer abnehmbaren Abdeckung (36) befindet.

8. Intravaginale Vorrichtung nach einem der Ansprüche 1-7, wobei der expandierbare Abschnitt (20) einen oder mehrere Sensoren (60) und eine oder mehrere andere elektrische Komponenten (62) umfasst.

9. Intravaginale Vorrichtung nach einem der Ansprüche 1-8, wobei der expandierbare Abschnitt (20) mindestens eines der Folgenden umfasst: eine Sendekomponente, eine Empfangskomponente, einen Ultraschallwandler, einen Schallwandler, einen Volumen- und/oder Fluiddurchflusssensor, einen optischen Sensor, einen Doppler-Sensor, eine Kamera, eine lichtemittierende Quelle und/oder einen Lateral-Flow-Testsensor.

10. Intravaginale Vorrichtung nach einem der Ansprüche 1-9, wobei sich der expandierbare Abschnitt (20) anfänglich innerhalb eines Applikators (12) befindet, der ein mit Schlitzen (19) ausgebildetes, öffenbares distales Ende (18) umfasst, und wobei ein Kolben (14) dazu angeordnet ist, sich in Bezug auf den Applikator (12) zu bewegen, wodurch eine Relativbewegung des expandierbaren Abschnitts (20) in Bezug auf den Applikator (12) bewirkt wird.

11. Intravaginale Vorrichtung nach einem der Ansprüche 1-10, ferner umfassend einen Auffangbeutel für vaginalen Ausfluss (50).

12. Intravaginale Vorrichtung nach Anspruch 11, wobei der Auffangbeutel für vaginalen Ausfluss (50) die Vorrichtung vollständig umschließt, um das Auffangen jeglicher Flüssigkeiten zu ermöglichen.

13. Intravaginale Vorrichtung nach Anspruch 11 oder 12, wobei der Auffangbeutel für vaginalen Ausfluss (50) ein antibakterielles Material umfasst oder eine Textur mit antibakteriellen Eigenschaften aufweist.

14. Intravaginale Vorrichtung nach einem der Ansprüche 11-13, wobei der Auffangbeutel für vaginalen Ausfluss (50) über ein Schließventil mit der Zugschnur (35) verbunden ist und wobei das Ventil den Auffangbeutel für vaginalen Ausfluss (50) schließt, wenn an der Zugschnur (35) gezogen wird.

## Revendications

1. Dispositif intravaginal (10), comprenant :
une partie extensible (20) comprenant une ou plusieurs entretoises (28) couplées de manière pivotante à un ou plusieurs bras de liaison (30), lesdits un ou plusieurs bras de liaison (30) étant couplés de manière pivotante à une tige (32), et lesdites une ou plusieurs entretoises (28) étant couplées de manière pivotante à un organe de base (34) couplé à ladite tige (32), lesdites une ou plusieurs entretoises (28) comprenant un groupe (28A) d'entretoises à expansion plus importante et un groupe (28B) d'entretoises à expansion plus faible ;
un mécanisme d'expansion d'entretoise couplé auxdites une ou plusieurs entretoises pour dilater lesdites une ou plusieurs entretoises (28) vers l'extérieur à partir de ladite tige (32), ledit groupe (28A) d'entretoises à expansion plus importante se dilatant davantage vers l'extérieur que ledit groupe (28B) d'entretoises à expansion plus faible ; et
une ficelle de traction (35) couplée à une partie de ladite partie extensible (20).

2. Dispositif intravaginal selon la revendication 1, dans lequel le mécanisme d'expansion est configuré pour faire coulisser le groupe (28A) d'entretoises à expansion plus importante et le groupe (28B) d'entretoises à expansion plus faible sur la tige (32).

3. Dispositif intravaginal selon la revendication 1 ou 2, dans lequel ledit groupe (28A) d'entretoises à expansion plus importante coulisse sur une plus grande distance le long de ladite tige (32) que ledit groupe (28B) d'entretoises à expansion plus faible.

4. Dispositif intravaginal selon l'une quelconque des revendications 1 à 3, dans lequel lesdites une ou plusieurs entretoises (28) comprennent une pluralité d'entretoises qui se dilatent vers l'extérieur en une forme non circulaire selon une expansion non uniforme.

5. Dispositif intravaginal selon l'une quelconque des revendications 1 à 4, dans lequel ledit mécanisme d'expansion d'entretoise comprend une première bague coulissante (40) couplée de manière pivotante audit groupe (28A) d'entretoises à expansion plus importante et agencée pour coulisser sur ladite tige (32), et une seconde bague coulissante (42) couplée de manière pivotante audit groupe (28B) d'entretoises à expansion plus faible et agencée pour coulisser sur ladite tige (32).

6. Dispositif intravaginal selon la revendication 5, dans lequel la première bague coulissante (40) comprend un ergot (44) reçu dans un canal (46) formé dans ladite seconde bague coulissante (42).

7. Dispositif intravaginal selon l'une quelconque des revendications 1 à 6, dans lequel ladite partie extensible (20) se trouve initialement à l'intérieur d'un cache amovible (36).

8. Dispositif intravaginal selon l'une quelconque des revendications 1 à 7, dans lequel ladite partie extensible (20) comprend un ou plusieurs capteurs (60) et un ou plusieurs autres composants électriques (62).

9. Dispositif intravaginal selon l'une quelconque des revendications 1 à 8, dans lequel ladite partie extensible (20) comprend au moins l'un parmi : un composant émetteur, un composant récepteur, un transducteur à ultrasons, un transducteur acoustique, un capteur volumétrique et/ou de débit de fluide, un capteur optique, un capteur Doppler, une caméra, une source émettrice de lumière et/ou un capteur de test à flux latéral.

10. Dispositif intravaginal selon l'une quelconque des revendications 1 à 9, dans lequel ladite partie extensible (20) se trouve initialement à l'intérieur d'un applicateur (12) comprenant une extrémité (18) distale pouvant s'ouvrir pourvue de fentes (19), et un piston (14) est agencé pour se déplacer par rapport audit applicateur (12), ce qui provoque un déplacement relatif de ladite partie extensible (20) par rapport audit applicateur (12).

11. Dispositif intravaginal selon l'une quelconque des revendications 1 à 10, comprenant en outre un sac (50) collecteur de sécrétions vaginales.

12. Dispositif intravaginal selon la revendication 11, dans lequel ledit sac (50) collecteur de sécrétions vaginales enveloppe entièrement le dispositif afin de permettre la collecte de tout fluide.

13. Dispositif intravaginal selon la revendication 11 ou 12, dans lequel ledit sac (50) collecteur de sécrétions vaginales comprend un matériau antibactérien ou présente une texture dotée de propriétés antibactériennes.

14. Dispositif intravaginal selon l'une quelconque des revendications 11 à 13, dans lequel ledit sac (50) collecteur de sécrétions vaginales est relié à la ficelle de traction (35) par l'intermédiaire d'une valve de fermeture et, lorsque la ficelle (35) est tirée, la valve ferme ledit sac (50) collecteur de sécrétions vaginales.
